(19) Europäisches Patentamt
European Patent Office
Office européen des brevets

(11) EP 3 683 803 A1

(12) EUROPEAN PATENT APPLICATION
published in accordance with Art. 153(4) EPC

(43) Date of publication:
22.07.2020 Bulletin 2020/30

(51) Int Cl.:
G21K 5/00 (2006.01)   G21K 5/04 (2006.01)
A61L 2/08 (2006.01)   B01J 19/12 (2006.01)

(21) Application number: 18856749.9

(22) Date of filing: 05.09.2018

(86) International application number:
PCT/JP2018/032843

(87) International publication number:
WO 2019/054245 (21.03.2019 Gazette 2019/12)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 12.09.2017 JP 2017174435

(71) Applicant: Hitachi Zosen Corporation
Osaka-shi, Osaka 559-8559 (JP)

(72) Inventors:
• TANAKA Ryuta
  Osaka-shi
  Osaka 559-8559 (JP)
• SAKAI Ichiro
  Osaka-shi
  Osaka 559-8559 (JP)
• NODA Takeshi
  Osaka-shi
  Osaka 559-8559 (JP)

(74) Representative: Engelhardt, Harald
Le Vrang & Engelhardt
Fliederstrasse 1
85139 Wettstetten (DE)

(54) **ELECTRON BEAM IRRADIATION DEVICE AND METHOD FOR MANUFACTURING SAME**

(57) An electron beam irradiation device (1) includes a vacuum chamber (2) having an electron beam generator (21) inside, a vacuum nozzle (3), and a window foil (4) on a tip of the vacuum nozzle (3). The electron beam irradiation device (1) further includes an outer pipe (7) surrounding the vacuum nozzle (3), a cooling-gas supply unit (9) that supplies cooling gas (C) into a coolant passage (8) formed between the vacuum nozzle (3) and the outer pipe (7), and a heat-conducting transmission foil (6) fitted to the window foil (4) and contacting the tip of the vacuum nozzle (3). The heat-conducting transmission foil (6) has a value of at least $63 \times 10^{-3}$, which is determined by dividing a thermal conductivity [W/(m·K)] by a density [kg/m$^3$], and a tip part of the vacuum nozzle (3) is made of a material having at least a thermal conductivity of copper.

FIG. 1

COOLING-GAS SUPPLY UNIT

EP 3 683 803 A1

## Description

Technical Field

The present invention relates to an electron beam irradiation device that emits an electron beam from the tip of a vacuum nozzle, and a method for manufacturing the same.

Background Art

An electron beam irradiation device including a vacuum nozzle features electron beam irradiation from the tip of the vacuum nozzle. The vacuum nozzle is inserted into the opening of a container or the like and emits an electron beam, thereby sterilizing the inner surface of the container. Such an electron beam irradiation device is used for sterilizing containers for foods and beverages or medical containers.

Containers for foods and beverages or medical containers are used in large quantity and thus high-volume sterilization is necessary. Hence, electron beam sterilization equipment for such sterilization typically includes a large number of electron beam irradiation devices (for example, see FIG. 11 in Patent Literature 1). In electron beam sterilization equipment, electron beam irradiation generates toxic gas and electromagnetic waves, for example, ozone gas, nitric acid gas, and X-rays and thus apparatuses for treating such gas and electromagnetic waves are provided. Thus, electron beam sterilization equipment typically has a large size and a complicated configuration.

This leads to the need for simple electron beam sterilization equipment, so that electron beam irradiation devices serving as the main components of electron beam sterilization equipment need to have simple configurations.

Citation List

Patent Literature

Patent Literature 1: Japanese Patent No. 5753047

Summary of Invention

Technical Problem

In an electron beam irradiation device described in Patent Literature 1, an exit window 8 (hereinafter, will be referred to as a window foil) is supported by a support 26 (hereinafter, will be referred to as a grid). In this configuration, the grid interferes with electron beam irradiation, thereby reducing the yields of emitted electron beams. Hence, to obtain required electron beam irradiation in the electron beam irradiation device, it is necessary to generate electron beams with high power. This also enhances heat generation on a window foil that allows the passage of electron beams with high power, so that a complicated cooling mechanism (see FIG. 1a in Patent Literature 1) that makes cooling gas impinge on the window foil directly is necessary for sufficiently cooling the window foil.

An object of the present invention is to provide an electron beam irradiation device that has a simple configuration and eliminates the need for a complicated configuration for cooling a window foil.

Solution to Problem

In order to solve the problem, an electron beam irradiation device according to a first invention includes:

a vacuum chamber;
an electron beam generator disposed in the vacuum chamber;
a vacuum nozzle connected to the vacuum chamber with air tightness so as to guide an electron beam from the electron beam generator;
a window foil that is disposed on a tip of the vacuum nozzle and allows the transmission of the electron beam from inside to outside of the vacuum nozzle;
an outer pipe surrounding an outer surface of the vacuum nozzle;
a cooling-gas supply unit that supplies cooling gas into a coolant passage formed as a clearance between the vacuum nozzle and the outer pipe; and
a heat-conducting transmission foil that is fitted to the window foil and is in contact with the tip of the vacuum nozzle, wherein the heat-conducting transmission foil is made of a material having a value of at least $63 \times 10^{-3}$, which is determined by dividing a thermal conductivity [W/(m·K)] by a density [kg/m$^3$], and

at least a tip part of the vacuum nozzle is made of a material having at least a thermal conductivity of copper.

An electron beam irradiation device according to a second invention, wherein the heat-conducting transmission foil in the electron beam irradiation device according to the first invention is made of beryllium, a carbon material, aluminum or silicon, or compounds thereof.

An electron beam irradiation device according to a third invention, wherein in the electron beam irradiation device according to one of the first and second inventions, one of the window foil and the heat-conducting transmission foil with lower corrosion resistance is disposed near the vacuum nozzle.

An electron beam irradiation device according to a fourth invention further includes, in the electron beam irradiation device according to one of the first to third inventions, an adhesive member between the tip of the vacuum nozzle and one of the heat-conducting transmission foil and the window foil.

A method for manufacturing an electron beam irradiation device according to a fifth invention is a method of manufacturing the electron beam irradiation device according to any one of the first to fourth inventions, the method including:

forming the laminated foil by fitting the heat-conducting transmission foil to the window foil;
placing the laminated foil on the tip of the vacuum nozzle; and
connecting the vacuum nozzle to the vacuum chamber,
wherein in the formation of the laminated foil, the window foil and the heat-conducting transmission foil are fitted to each other by pressure welding.

Advantageous Effects of Invention

According to the electron beam irradiation device and the method for manufacturing the same, the window foil is sufficiently cooled, thereby eliminating the need for a complicated configuration for cooling the window foil. This can achieve a simple configuration.

Brief Description of Drawings

[FIG. 1] FIG. 1 is a schematic longitudinal section illustrating an electron beam irradiation device according to an embodiment of the present invention.
[FIG. 2] FIG. 2 is an enlarged view illustrating a principal part of the electron beam irradiation device.
[FIG. 3] FIG. 3 is an exploded perspective view illustrating a longitudinal section of a laminated foil and an adhesive member in the electron beam irradiation device.
[FIG. 4] FIG. 4 is a schematic longitudinal section illustrating the electron beam irradiation device according to an example of the present invention.
[FIG. 5] FIG. 5 is an enlarged view illustrating a tip part of a vacuum nozzle as a principal part of the electron beam irradiation device.

Description of Embodiment

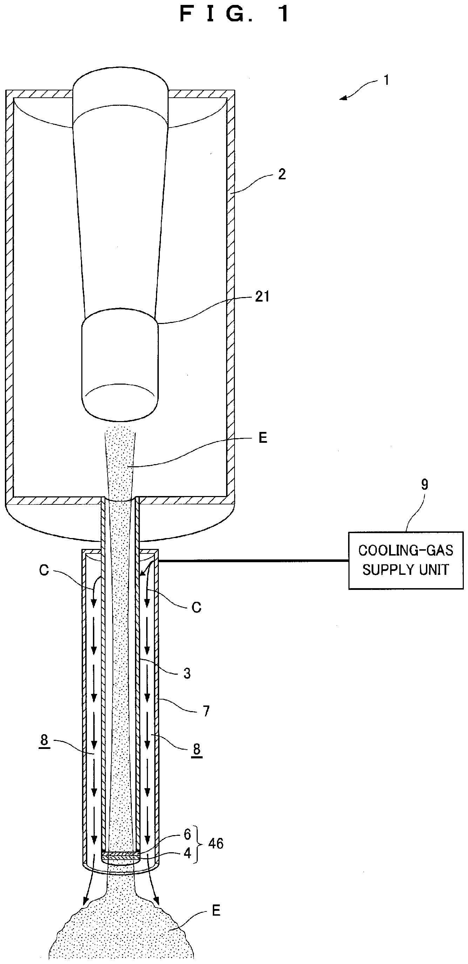

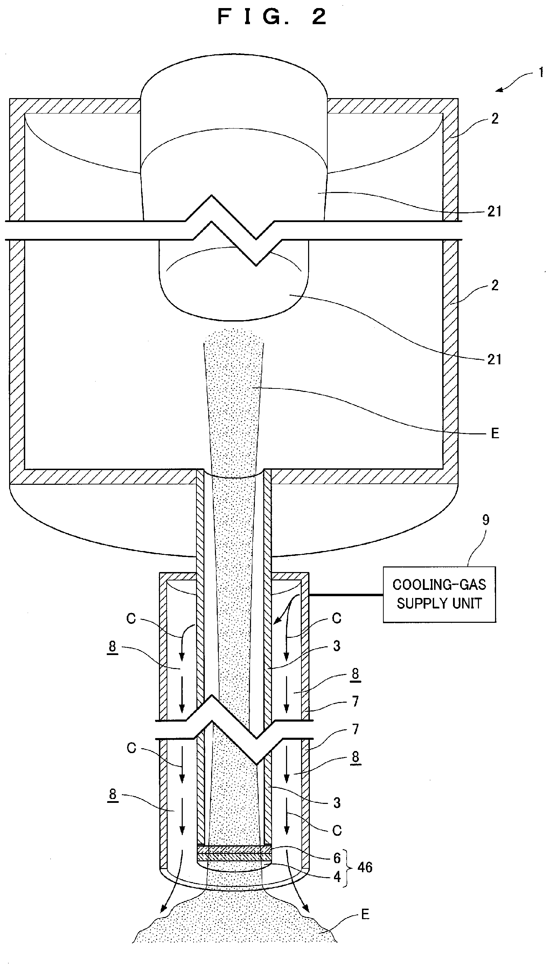

Referring to FIGS. 1 and 2, an electron beam irradiation device according to an embodiment of the present invention will be described below.

As illustrated in FIG. 1, an electron beam irradiation device 1 includes a vacuum chamber 2 in which an electron beam generator 21 is disposed, a vacuum nozzle 3 that is connected to the vacuum chamber 2 with airtightness so as to guide an electron beam E from the electron beam generator 21, and a window foil 4 that is disposed on the tip of the vacuum nozzle 3 and allows the transmission of the electron beam E from the inside of the vacuum nozzle 3 to the outside.

The vacuum chamber 2 is evacuated in order to accelerate the electron beam E from the electron beam generator 21. Power for generating the electron beam E is supplied to the electron beam generator 21 from, for example, a power supply disposed outside the vacuum chamber 2. The power supply is not illustrated. The vacuum nozzle 3 is evacuated along with the vacuum chamber 2. The window foil 4 seals the tip of the vacuum nozzle 3 and radiates the transmitted electron beam E to the outside of the vacuum nozzle 3. The transmission of the electron beam E heats the window foil 4. For cooling the window foil 4, the electron beam irradiation device 1 is configured as follows:

As illustrated in FIG. 2, the electron beam irradiation device 1 further includes an outer pipe 7 surrounding the outer surface of the vacuum nozzle 3, a cooling-gas supply unit 9 that supplies cooling gas C (e.g., air) into a coolant passage 8, that is, a clearance 8 between the vacuum nozzle 3 and the outer pipe 7, and a heat-conducting transmission foil 6 that is fit with a low density and a high thermal conductivity to the window foil 4 and is in contact with the tip of the vacuum nozzle 3. Additionally, at least a tip part of the vacuum nozzle 3 is made of a thermal conductive material (a material having at least a thermal conductivity of copper). In other words, in the electron beam irradiation device 1, heat from the window foil 4 is quickly transmitted to the tip part of the vacuum nozzle 3 by the heat-conducting transmission foil 6 and the transmitted heat is quickly transferred to the cooling gas C supplied into the clearance 8 between the vacuum nozzle 3 and the outer pipe 7. This configuration sufficiently cools the window foil 4 and thus eliminates the need for makeing the cooling gas C impinge on the window foil 4 directly through the outer pipe 7. Thus, the outer pipe 7 has a fixed internal diameter (including manufacturing errors such as a tolerance). The tip part of the vacuum nozzle 3 is made of the thermal conductive material (a material having at least a thermal conductivity of copper) and may have any length as long as heat from the window foil 4 is quickly transferred to the cooling gas C. For example, the tip part is at least four times longer than the internal diameter of the vacuum nozzle 3. If the tip part is at least four times longer than the internal diameter of the vacuum nozzle 3, an area exposed to the cooling gas C on the tip part is at least 16 times larger than the area of the window foil 4 (to be accurate, the area of one side inside the vacuum nozzle 3), that is, the tip part is so wide that heat transmitted from the window foil 4 to the tip part is quickly transferred to the cooling gas C.

The heat-conducting transmission foil 6 having a low density and a high thermal conductivity is foil made of a material having a value of at least $63 \times 10^{-3}$, which is determined by dividing a thermal conductivity [W/(m·K)] by a density [kg/m$^3$]. The material satisfies Expression (1) below.

$$\text{Thermal conductivity } [\text{W/(m·K)}]/\text{density } [\text{kg/m}^3] \geq 63 \times 10^{-3} \dots (1)$$

In addition to quick heat transfer with a high thermal conductivity from the window foil 4 to the tip part of the vacuum nozzle 3, the heat-conducting transmission foil 6 hardly generates heat even when the electron beam E is transmitted due to the low density. Examples of specific materials of the heat-conducting transmission foil 6, that is, examples of materials satisfying Expression (1) include beryllium, carbon materials (e.g., graphite, graphene, or a carbon nanotube), aluminum or silicon, or compounds thereof. Hereinafter, the window foil 4 and the heat-conducting transmission foil 6 fit onto the window foil 4 will be collectively referred to as a laminated foil 46.

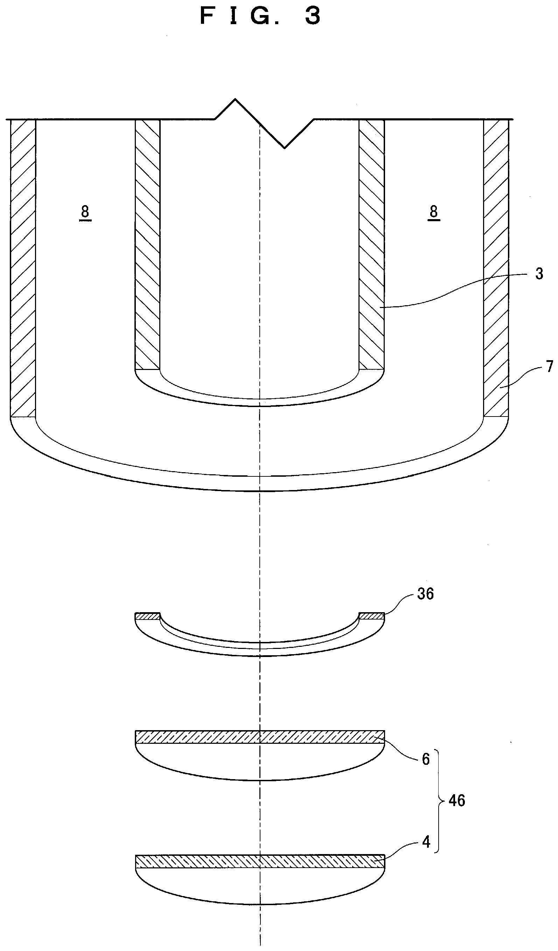

The tip of the vacuum nozzle 3 and the heat-conducting transmission foil 6 may be hardly bonded to each other depending on the materials of the vacuum nozzle 3 and the heat-conducting transmission foil 6. In this case, as illustrated in FIG. 3, an exploded perspective view of a longitudinal section, an adhesive member 36 that is highly adhesive to both of the tip of the vacuum nozzle 3 and the heat-conducting transmission foil 6 may be interposed therebetween. In order not to hinder the passage of the electron beam E, the adhesive member 36 is ring-shaped. Also in the case where the adhesive member 36 is interposed, the heat-conducting transmission foil 6 and the tip of the vacuum nozzle 3 are preferably brought into contact with each other in order to quickly transmit heat from the heat-conducting transmission foil 6 to the tip part of the vacuum nozzle 3. In addition to the configuration of FIG. 3, the adhesive member 36 highly adhesive to both of the tip of the vacuum nozzle 3 and the window foil 4 may be interposed so as to indirectly bond the tip of the vacuum nozzle 3 and the window foil 4.

In view of quick transfer of heat from the heat-conducting transmission foil 6 to the tip part of the vacuum nozzle 3, it is preferably smooth the contact surfaces of the heat-conducting transmission foil 6 and the tip of the vacuum nozzle 3 (e.g., arithmetic mean roughness Ra ≤ 0.4). Naturally, the smoothed contact surfaces achieve connection with firmer air tightness as well as quick transfer of heat.

A method for manufacturing the electron beam irradiation device 1 will be described below.

The method for manufacturing the electron beam irradiation device 1 includes a laminated foil forming step in which the heat-conducting transmission foil 6 is fitted to the window foil 4 so as to form the laminated foil 46, a laminated foil placing step in which the laminated foil 46 is placed on the tip of the vacuum nozzle 3, and a connecting step in which the vacuum nozzle 3 is connected to the vacuum chamber 2. In these steps, the laminated foil placing step follows the laminated foil forming step. The order of the connecting step is not limited.

In the laminated foil forming step, the window foil 4 and the heat-conducting transmission foil 6 are fitted to each other by pressure welding. In this case, pressure welding is a bonding method for applying heat and/or a pressure to the adjoining contact surfaces of the window foil 4 and the heat-conducting transmission foil 6 so as to metallically fuse the atoms of the window foil 4 and the heat-conducting transmission foil 6. The bonding method is, for example, diffusion bonding. The laminated foil 46 is formed by pressure welding, so that the window foil 4 and the heat-conducting transmission foil 6 are firmly fitted to each other. Thus, the laminated foil 46 is unlikely to be broken even under severe conditions such as a high pressure applied by sealing the evacuated vacuum nozzle 3 and heat generated by the transmitted electron beam E.

In the laminated foil placing step, in order to place the formed laminated foil 46 on the tip of the vacuum nozzle 3, the laminated foil 46 is bonded to the tip of the vacuum nozzle 3 by, for example, brazing.

In the connecting step, the vacuum chamber 2 and the vacuum nozzle 3 are directly connected to each other or indirectly connected to each other via a flange or the like (not illustrated). The vacuum chamber 2 in the connecting step may contain necessary devices such as the electron beam generator 21 and a power supply or the necessary devices may be disposed in the vacuum chamber 2 after the connecting step.

The operations of the electron beam irradiation device 1 will be described below.

First, power is supplied to the electron beam generator 21 from the power supply (not illustrated), so that the electron beam E is generated from the electron beam generator 21 as illustrated in FIGS. 1 and 2. The electron beam E is accelerated in the vacuum chamber 2 and the vacuum nozzle 3, is guided into the vacuum nozzle 3, and then passes through the laminated foil 46. This mainly heats the window foil 4. Heat from the window foil 4 is immediately transmitted to the tip part of the vacuum nozzle 3 through the heat-conducting transmission foil 6. The transmitted heat is quickly transferred to the cooling gas C supplied to the clearance 8 between the vacuum nozzle 3 and the outer pipe 7. Thus, the window foil 4 is sufficiently cooled.

In this way, the window foil 4 is sufficiently cooled in the electron beam irradiation device 1, thereby eliminating the need for a complicated configuration for cooling the window foil 4. This can achieve a simple configuration.

Moreover, the heat-conducting transmission foil 6 is made of the material that is easily obtained, such as beryllium, carbon materials, aluminum or silicon, or compounds thereof, achieving a simpler configuration.

Furthermore, the adhesive member 36 placed as illustrated in FIG. 3 firmly bonds the heat-conducting transmission foil 6 to the tip of the vacuum nozzle 3, thereby improving the durability.

Additionally, according to the method for manufacturing the electron beam irradiation device 1, the window foil 4 and the heat-conducting transmission foil 6 are firmly bonded to each other by contact bonding, thereby further improving durability.

In the present embodiment, at least the tip part of the vacuum nozzle 3 is made of the thermal conductive material (a material having at least a thermal conductivity of copper). The thermal conductive material is used to transfer heat from the window foil 4 to the cooling gas C at least on the tip part of the vacuum nozzle 3. Thus, the part of the thermal conductive material (a material having at least a thermal conductivity of copper) in the vacuum nozzle 3 is preferably exposed as large an area as possible to the cooling gas C. In other words, the part of the thermal conductive material (a material having at least a thermal conductivity of copper) in the vacuum nozzle 3 preferably extends so as to be entirely surrounded by the outer pipe 7. As illustrated in FIGS. 1 and 2, the overall vacuum nozzle 3 is made of the conductive material (a material having at least a thermal conductivity of copper). This configuration is further preferable because heat is transferred from a part not surrounded by the outer pipe 7 in addition to the cooling gas C.

In the embodiment, the heat-conducting transmission foil 6 in the laminated foil 46 is illustrated near the vacuum nozzle 3. The window foil 4 may be disposed near the vacuum nozzle 3. In the laminated foil 46, the foil having lower corrosion resistance is disposed near the vacuum nozzle 3 and thus the laminated foil 46 becomes less corrosive, further improving the durability.

In the embodiment, the cooling-gas supply unit 9 supplies the cooling gas C. The cooling-gas supply unit 9 may supply and collect the cooling gas C (in other words, the cooling gas C is circulated). The cooling-gas supply unit 9 may be replaced with a cooling-liquid supply unit that supplies and circulates a cooling liquid (water or oil). This configuration is further preferable because the cooling liquid efficiently collects heat from the tip part.

Additionally, in the embodiment, the tip part of the vacuum nozzle 3 has a flat outer surface. The outer surface of the tip part may have a large number of grooves so as to increase the area of the tip part exposed to the cooling gas C. In particular, the grooves are more preferably formed perpendicularly to the axis of the vacuum nozzle 3 (that is, a large number of circumferential grooves) because the cooling gas C efficiently collects heat from the tip part. As a matter of course, the grooves may be replaced with a large number of projections. In order to more efficiently transfer heat from the tip part to the cooling gas C, a cooling fin may be provided on the tip part.

In the embodiment, the shape and thickness of the heat-conducting transmission foil 6 were not specifically described. The heat-conducting transmission foil 6 may have any shape and thickness as long as heat is quickly transferred from the window foil 4 to the tip part of the vacuum nozzle 3 and heat is hardly generated even when the electron beam E is transmitted due to the low density. For example, the heat-conducting transmission foil 6 is preferably so thick that heat other than heat transferred through the window foil 4 exposed to the atmosphere (other than convective heat transfer and thermal radiation) is completely transmitted to the tip part of the vacuum nozzle 3. Specifically, if the window foil 4 is a titanium foil having a thickness of 5 $\mu$m and the heat-conducting transmission foil 6 is an aluminum foil having a thickness of 8 $\mu$m, at least about 99% of heat generated on the titanium foil by the transmission of the electron beam E is transmitted to the tip part of the vacuum nozzle 3 so as to cool the titanium foil. This leaves only a small amount of heat on the titanium foil and thus the heat is sufficiently transferred through the titanium foil exposed to the atmosphere. Hence, a temperature increase on the titanium foil is suppressed. For comparison, in the related art where the heat-conducting transmission foil 6 is not provided (in other words, only the window foil 4 is provided) and the window foil 4 is a titanium foil having a thickness of 10 $\mu$m, about 75% of heat generated on the titanium foil by the transmission of the electron beam E is transmitted to the tip part of the vacuum nozzle 3 so as to cool the titanium foil. This leaves a larger amount of heat on the titanium foil and thus the heat is not sufficiently transferred through the titanium foil exposed to the atmosphere. Hence, a temperature increase on the titanium foil is not suppressed.

A simulation was conducted to confirm the effect of the embodiment. First, conditions were set as follows: the titanium foil and the aluminum foil had the thicknesses described in the embodiment, the vacuum nozzle 3 had an internal diameter of 4 mm, and the electron beam E was generated so as to heat the tip and base of the vacuum nozzle 3 to temperatures of 700 K and 400 K, respectively. As a result, in the case of a foil including a titanium foil (5 μm thick) and an aluminum foil (8 μm thick) on the tip of the vacuum nozzle 3, the titanium foil was cooled by no less than 7.6 W without forced air-cooling. In contrast, in the case of a foil only including a titanium foil (10 μm thick) on the tip of the vacuum nozzle 3, the titanium foil was cooled only by 1.2 W with forced air-cooling. Thus, it is assumed that the foil including the window foil 4 and the heat-conducting transmission foil 6, that is, the laminated foil 46 on the tip of the vacuum nozzle 3 is more resistant to the electron beam E having a large current.

Example

According to a more specific example of the embodiment, the electron beam irradiation device 1 will be described below based on the accompanying drawings. Configurations omitted in the embodiment will be mainly discussed. The same configurations as those of the embodiment are indicated by the same reference numerals and the explanation thereof is omitted.

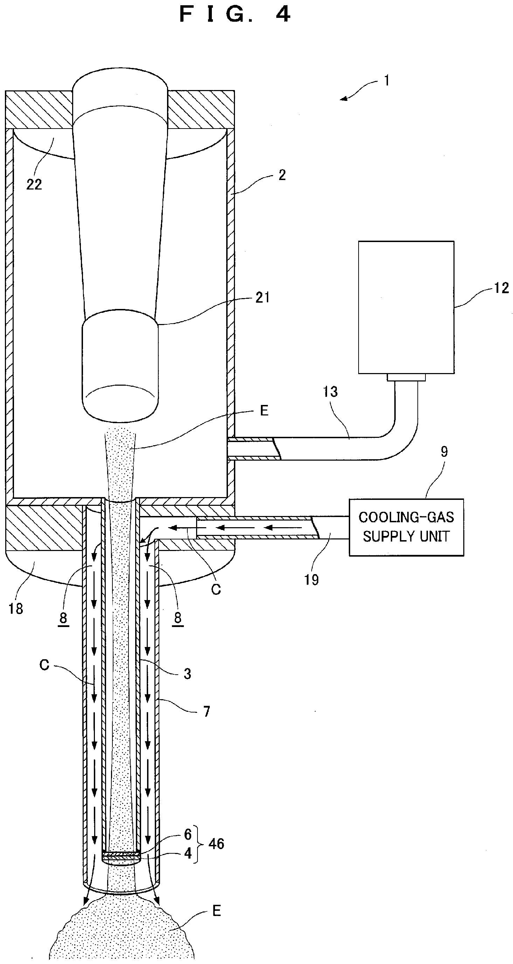

As illustrated in FIG. 4, the electron beam irradiation device 1 according to the example includes a vacuum pump 12 for evacuating the vacuum chamber 2 and the vacuum nozzle 3, and a vacuum L-shaped pipe 13 connecting the vacuum chamber 2 and the vacuum pump 12. Moreover, the electron beam irradiation device 1 includes an external flange 18 that fixes the vacuum nozzle 3 to the vacuum chamber 2 and guides the cooling gas C into the coolant passage 8, and a coolant pipe 19 connecting the external flange 18 and the cooling-gas supply unit 9.

The vacuum chamber 2 includes an internal flange 22 for fixing the electron beam generator 21. The internal flange 22 is disposed on the opposite end of the vacuum chamber 2 from the vacuum nozzle 3. The vacuum nozzle 3 is made of a copper alloy. The vacuum pump 12 is capable of setting the interiors of the vacuum chamber 2 and the vacuum nozzle 3 at a degree of vacuum (high vacuum to ultrahigh vacuum) suitable for accelerating the electron beam E. The vacuum L-shaped pipe 13 is positioned so as to separate the vacuum pump 12 from the electron beam E in the vacuum chamber 2 while locating the axis of the vacuum pump 12 in parallel with the axis of the vacuum chamber 2. Thus, even if a magnetic field is generated by the vacuum pump 12, the influence of the magnetic field on the electron beam E can be reduced.

The external flange 18 stably holds the vacuum nozzle 3 cantilevered from the vacuum chamber 2 and simplifies a structure from the coolant pipe 19 to the coolant passage 8, that is, a structure that guides the cooling gas C from the coolant pipe 19 to the coolant passage 8. The coolant pipe 19 is not particularly limited but is preferably short in length such that the cooling gas C does not collect heat other than heat from the vacuum nozzle 3, which is unnecessary heat.

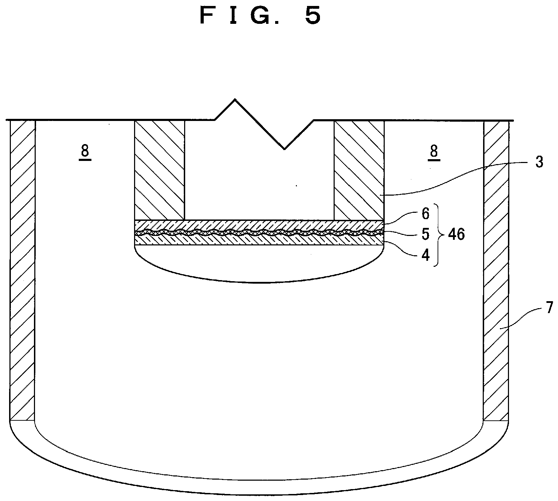

As illustrated in FIG. 5, the window foil 4 is not limited as long as the window foil 4 allows the transmission of the electron beam E and is resistant to a high atmospheric pressure applied by sealing the evacuated vacuum nozzle 3. The window foil 4 is, for example, a titanium foil having a uniform thickness of about 1 μm to 10 μm (preferably about 3 μm to 5 μm). The heat-conducting transmission foil 6 is, for example, an aluminum foil similarly having a uniform thickness of about 2 μm to 20 μm (preferably about 5 μm to 15 μm). The laminated foil 46 including the window foil 4 and the heat-conducting transmission foil 6 (strictly speaking, also including a boundary layer 5) is quite thin and always receives a high atmospheric pressure and thus is easily broken by an unexpected collision or the like. Thus, the structure is formed such that the laminated foil 46 disposed on the tip of the vacuum nozzle 3 is sufficiently surrounded by the outer pipe 7, that is, the tip of the outer pipe 7 projects out of the tip of the vacuum nozzle 3. The projection has any length as long as the laminated foil 46 is protected by the outer pipe 7. The projection is not shorter than, for example, the internal diameter of the vacuum nozzle 3.

A method for manufacturing the electron beam irradiation device 1 will be described below.

As the laminated foil forming step, the window foil 4 and the heat-conducting transmission foil 6 are fitted to each other by diffusion bonding. As illustrated in FIG. 5, through the diffusion bonding, the boundary layer 5 is formed between the window foil 4 and the heat-conducting transmission foil 6 by chemical bonding between the material of the window foil 4 and the material of the heat-conducting transmission foil 6. The boundary layer 5 has any thickness as long as the window foil 4 and the heat-conducting transmission foil 6 are fitted to each other. Thus, the time for diffusion bonding is set such that the boundary layer 5 is sufficiently thick.

The operations of the electron beam irradiation device 1 will be described below.

First, as illustrated in FIG. 4, the vacuum pump 12 evacuates the vacuum chamber 2 and the vacuum nozzle 3 to a degree of vacuum (high vacuum to ultrahigh vacuum) suitable for accelerating the electron beam E. Subsequently, power is supplied to the electron beam generator 21 from the power supply (not illustrated), so that the electron beam E is generated from the electron beam generator 21. The electron beam E is accelerated in the vacuum chamber 2 and the vacuum nozzle 3, is guided into the vacuum nozzle 3, and then passes through the laminated foil 46. This mainly heats the window foil 4. Heat from the window foil 4 is immediately transmitted to the tip part of the vacuum nozzle 3 through the heat-conducting transmission foil 6. Since the vacuum nozzle 3 is entirely made of a copper alloy, heat from the heat-conducting transmission foil 6 is transmitted over the vacuum nozzle 3. Most of the heat transmitted to the vacuum nozzle 3 is quickly transferred to the cooling gas C supplied into the clearance 8 between the vacuum nozzle 3 and the outer pipe 7. Thus, the window foil 4 is sufficiently cooled.

In addition to the effect of the electron beam irradiation device 1 according to the embodiment, the electron beam irradiation device 1 of the present example achieves the following effect: The durability is further improved for the following reasons. Firstly, as illustrated in FIG. 5, the laminated foil 46 of the present example includes the boundary layer 5 firmly bonding the window foil 4 and the heat-conducting transmission foil 6 and is surrounded by the outer pipe 7. Secondly, as illustrated in FIG. 4, the vacuum nozzle 3 is fixed to the external flange 18 so as to be connected to the vacuum chamber 2.

In the example, the detail of the vacuum nozzle 3 was specifically determined and a simulation result was obtained as follows: The vacuum nozzle 3 was 150 mm in length, 4 mm in internal diameter, and 1 mm in thickness and was made of copper. The electron beam E was generated so as to heat the tip and base of the vacuum nozzle 3 to temperatures of 400 K and 300 K, respectively. In this case, a heat quantity of no less than 7.48 W was transferred and sufficient cooling was achieved. For comparison, under the same conditions except that the vacuum nozzle 3 was replaced with a stainless vacuum nozzle, only a heat quantity of 1.64 W was transferred and resulted in insufficient cooling.

The embodiment and the example are merely exemplary and are not restrictive in all the aspects. The scope of the present invention is not indicated by the foregoing description but the claims. The scope of the present invention is intended to include meanings equivalent to the claims and all changes in the scope. From among the configurations described in the embodiment and the example, the configurations other than that described as a first invention in "Solution to Problem" are optional and thus can be deleted and changed as appropriate.

## Claims

1.  An electron beam irradiation device comprising:

    a vacuum chamber;
    an electron beam generator disposed in the vacuum chamber;
    a vacuum nozzle connected to the vacuum chamber with air tightness so as to guide an electron beam from the electron beam generator;
    a window foil that is disposed on a tip of the vacuum nozzle and allows the transmission of the electron beam from inside to outside of the vacuum nozzle;
    an outer pipe surrounding an outer surface of the vacuum nozzle;
    a cooling-gas supply unit that supplies cooling gas into a coolant passage formed as a clearance between the vacuum nozzle and the outer pipe; and
    a heat-conducting transmission foil that is fitted to the window foil and is in contact with the tip of the vacuum nozzle,
    wherein the heat-conducting transmission foil is made of a material having a value of at least $63 \times 10^{-3}$, which is determined by dividing a thermal conductivity [W/(m·K)] by a density [kg/m$^3$], and
    at least a tip part of the vacuum nozzle is made of a material having at least a thermal conductivity of copper.

2.  The electron beam irradiation device according to claim 1, wherein the heat-conducting transmission foil is made of beryllium, a carbon material, aluminum or silicon, or compounds thereof.

3.  The electron beam irradiation device according to one of claims 1 and 2, wherein one of the window foil and the heat-conducting transmission foil with lower corrosion resistance is disposed near the vacuum nozzle.

4.  The electron beam irradiation device according to one of claims 1 and 2, further comprising an adhesive member between the tip of the vacuum nozzle and one of the heat-conducting transmission foil and the window foil.

5.  A method for manufacturing the electron beam irradiation device according to one of claims 1 and 2, comprising:

    forming the laminated foil by fitting the heat-conducting transmission foil to the window foil;
    placing the laminated foil on the tip of the vacuum nozzle; and
    connecting the vacuum nozzle to the vacuum chamber,

wherein in the formation of the laminated foil, the window foil and the heat-conducting transmission foil are fitted to each other by pressure welding.

# F I G . 1

COOLING-GAS
SUPPLY UNIT

# F I G. 2

COOLING-GAS
SUPPLY UNIT

# F I G. 3

# FIG. 4

COOLING-GAS
SUPPLY UNIT

# F I G. 5

## INTERNATIONAL SEARCH REPORT

| International application No. |
|---|
| PCT/JP2018/032843 |

### A. CLASSIFICATION OF SUBJECT MATTER
Int.Cl. G21K5/00(2006.01)i, G21K5/04(2006.01)i, A61L2/08(2006.01)i, B01J19/12(2006.01)i

According to International Patent Classification (IPC) or to both national classification and IPC

### B. FIELDS SEARCHED

Minimum documentation searched (classification system followed by classification symbols)
Int.Cl. G21K5/00(2006.01)i, G21K5/04(2006.01)i, A61L2/08(2006.01)i, B01J19/12(2006.01)i, B65B55/08(2006.01)i

Documentation searched other than minimum documentation to the extent that such documents are included in the fields searched
| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2014/175065 A1 (HITACHI ZOSEN CORP.) 30 October 2014, entire text, all drawings & US 2016/0064111 A1, entire text, all drawings & EP 2991078 A1 | 1–5 |
| A | JP 6068693 B1 (HAMAMATSU PHOTONICS K.K.) 25 January 2017, entire text, all drawings & JP 2017-122691 A & WO 2017/119180 A1 & TW 201735053 A & CN 108463858 A | 1–5 |
| A | JP 2016-176943 A (TETRA LAVAL HOLDINGS & FINANCE S.A.) 06 October 2016, entire text, all drawings & US 2015/0028220 A1, entire text, all drawings & US 2016/0307724 A1, entire text, all drawings & WO 2012/074453 A1 & CN 103229269 A & CN 106409637 A | 1–5 |

☒ Further documents are listed in the continuation of Box C.  ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 November 2018 (21.11.2018) | 04 December 2018 (04.12.2018) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2018/032843

C (Continuation).   DOCUMENTS CONSIDERED TO BE RELEVANT

| Category* | Citation of document, with indication, where appropriate, of the relevant passages | Relevant to claim No. |
|---|---|---|
| A | JP 57-158600 A (SIEMENS AG) 30 September 1982, entire text, all drawings & EP 0059249 A2, entire text, all drawings & DE 3108006 A1 | 1-5 |
| A | JP 2014-134548 A (HITACHI ZOSEN CORP.) 24 July 2014, entire text, all drawings & JP 2009-526971 A & JP 2013-224941 A & JP 5438325 B2 & US 2008/0073549 A1, entire text, all drawings & US 2010/0247373 A1, entire text, all drawings & US 2012/0294758 A1, entire text, all drawings & WO 2007/095205 A2 & CN 101416255 A | 1-5 |
| A | JP 2009-35330 A (KRONES AG) 19 February 2009, entire text, all drawings & JP 2012-6664 A & US 2009/0045350 A1, entire text, all drawings & EP 1982920 A1 & EP 1982921 A1 & EP 2325089 A1 & CN 101310773 A | 1-5 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5753047 B